Europäisches Patentamt

European Patent Office

Office européen des brevets

(19)

(11) Numéro de publication : **0 022 019 B2**

(12) **NOUVEAU FASCICULE DE BREVET EUROPEEN**

(45) Date de publication du nouveau fascicule du brevet : **21.11.91 Bulletin 91/47**

(51) Int. Cl.⁵ : **A23J 3/00, A61K 37/02**

(21) Numéro de dépôt : **80400946.2**

(22) Date de dépôt : **24.06.80**

(54) Hydrolysat enzymatique total de protéines de lactosérum, obtention et applications.

(30) Priorité : **26.06.79 FR 7916483**

(43) Date de publication de la demande :
**07.01.81 Bulletin 81/01**

(45) Mention de la délivrance du brevet :
**28.09.83 Bulletin 83/39**

(45) Mention de la décision concernant
l'opposition :
**21.11.91 Bulletin 91/47**

(84) Etats contractants désignés :
**BE CH DE FR GB IT LI LU NL SE**

(56) Documents cités :
**US-A- 2 180 637**
**US-A- 3 950 547**
**CHEMICAL ABSTRACTS, vol. 81, no. 3, 22 juillet 1974, abrégé 11969w, page 218 COLUMBUS, OHIO (US)**
**Analytical Biochemistry, vol. 22 (1968), pp. 161-165 (W.F.Blatt et al.)**
**Biochem. J., vol. 91 (1964), pp. 222-233 (Andrews)**

(73) Titulaire : **INSTITUT NATIONAL DE LA RECHERCHE AGRONOMIQUE (INRA)
149, rue de Grenelle
F-75341 Paris Cedex 07 (FR)**

(72) Inventeur : **Maubois, Jean-Louis
La Barre Guibourg
F-35740 Pace (FR)**
Inventeur : **Roger, Loic**
**27 Rue de Brest
F-35000 Rennes (FR)**
Inventeur : **Brulé, Gérard**
**5 Cours de Lisbonne
F-35100 Rennes (FR)**
Inventeur : **Piot, Michel**
**10 Rue du Bourdonnais
F-35000 Rennes (FR)**

(74) Mandataire : **Phélip, Bruno et al
c/o Cabinet Harlé & Phélip 21, rue de La Rochefoucauld
F-75009 Paris (FR)**

EP 0 022 019 B2

**Description**

Hydrolysat enzymatique total de protéines de lactosérum, obtention et applications

L'invention concerne le domaine du traitement du lactosérum. Elle a plus particulièrement pour objet l'obtention de produits résultant de l'hydrolyse des protéines contenues dans ledit lactosérum. Elle concerne notamment un procédé d'hydrolyse enzymatique, mettant en oeuvre, à titre d'enzyme, une enzyme capable de protéolyser la totalité des protéines du lactosérum, et de préférence la pancréatine. L'invention concerne également les applications des produits ainsi obtenus, en particulier comme aliments devant répondre à des besoins spécifiques nutritionnels.

Le lactosérum est un sous-produit bien connu de l'industrie laitière. Sa composition est approximativement celle d'un lait écrémé, privé de sa caséine. On obtient un lactosérum de type acide en acidifiant le lait soit par addition d'un acide inorganique soit par production d'acide lactique (ensemencement du lait par des ferments lactiques) à un pH proche du point isoélectrique des caséines. Le lactosérum est récupéré après séparation du caillé. L'addition de présure au lait provoque également la floculation de la caséine. Le lactosérum obtenu après synérèse est dénommé lactosérum-présure. Si la floculation a lieu au pH du lait ou à un pH légèrement inférieur mais au-dessus de 5,8—6,0, le lactosérum est dénommé lactosérum doux.

On définit donc le lactosérum par rapport à la nature de la coagulation du lait. Dans l'industrie fromagère, la plupart des lactosérums sont en fait des lactosérums mixtes où l'un des procédés de coagulation est prédominant par rapport à l'autre. Les lactosérums doux sont issus des fabrications de pâtes pressées cuites ou non (Emmental, Gruyère, Cheddar, Cantal, Saint-Paulin). Les lactosérums acides sont issus principalement des fabrications de pâtes fraîches et des caséineries. On trouve également toute un variété de lactosérums mixtes provenant de la fabrication de la plupart des pâtes molles et des pâtes persillées. La composition des lactosérums pourra donc varier dans d'assez larges limites selon le lait de départ et la technologie fromagère utilisée. Tous les lactosérums contiennent des éléments minéraux, des matières grasses, une certaine quantité d'acide lactique, d'enzymes coagulantes, la fraction la plus intéressante étant bien évidemment la fraction azotée. C'est elle qui est en effet essentiellement constituée par les protéines solubles du lait possédant une valeur biologique élevée.

Les trois destinations traditionnelles des lactosérums étaient l'épandage, le rejet en rivière et l'alimentation animale. On a déjà proposé des traitements industriels du lactosérum, tels que la concentration et le séchage, pour diminuer les coûts de transport, de stockage et de conservation. De nouvelles technologies permettent de séparer, de concentrer et de purifier sélectivement les composants du lactosérum et de modifier leurs caractéristiques physico-chimiques tout en maintenant et même en améliorant la qualité nutritionnelle. De telles techniques permettent d'aboutir à des produits originaux et variés tant sur le plan nutritionnel que technologique. Parmi ces techniques, on peut citer la déminéralisation, la filtration moléculaire. La différence de taille moléculaire qui existe entre les protéines et la matière grasse d'une part et les lactoses et les sels minéraux d'autre part rend possible leur séparation par filtration, en particulier par ultrafiltration. On obtient alors une solution enrichie en protéines qui constitue la fraction noble du lactosérum et une solution essentiellement constituée par du lactose et les sels minéraux, dénommée "jus lactosé".

Bien qu'en valeur relative, les protéines représentent une faible part de la matière sèche du lactosérum (inférieure à 12%) elles sont l'attrait principal dans la valorisation de ce sous-produit. La fraction protéique essentiellement constituée par les protéines solubles du lait, β-lactoglobuline, α-lactalbumine, sérum-albumine et imunoglobulines est intéressante en raison de sa valeur nutritionnelle et de ses propriétés fonctionnelles.

On trouvera des indications intéressantes sur les protéines du lait dans l'ouvrage McKENZIE, H. A. (1971)—Milk proteins, Vols. 1 et 2. Academic Press New York, Les concentrés protéiques issus des lactosérums ont une valeur nutritionnelle élevée: voir par exemple les articles de FORSUM, E. (1974) Nutritional evaluation of whey protein concentrates and their fractions. J. of Dairy Sc., 57, (6), 665—670. FORSUM, E. (1975)—Whey proteins for food and feed supplement. Protein nutritional quality of foods and feeds, part II 433—170, Marcel Bekker Ed., I.N.C. New York. FORSUM, E. (1975)—Effect of dietary lactose on nitrogen utilization of a whey protein concentrate and its corresponding amino acid a mixture. Nutrition Reports International, 11, (5) 419—428. FORSUM, E. (1977)—Use of a whey protein concentrate as a supplement to maize, rice and potatoes. A chemical and biological evaluation using growing rats. J. of Nutrition, 105, (2), 147—153.

Pour juger de la valeur nutritionnelle d'une protéine ou d'un aliment azoté protéique, on compare sa composition en acides aminés essentiels à celle d'une protéine de référence. Quelle que soit la protéine de référence choisie, la composition des protéines de lactosérum est bien équilibrée. On constate notamment une teneur élevée en lysine, thréonine, et tryptophane, une teneur en cystéine élevée par rapport au lait en une faible teneur en phénylalanine et en méthionine. On sait par ailleurs que la seule présence dans l'aliment d'un acide aminé ne témoigne pas forcément de son utilisation évidente par l'organisme. Le traitement auquel a été soumis le produit avant l'évaporation de celui-ci peut avoir partiellement rendu indisponible un certain nombre d'acides aminés. Cette constatation a été faite: voir CHEFTEL, C, et CHEFTEL, H. (1976)—Brunissement non enzymatique. Biochimie et Technologie des aliments, vol. 1, *3*, 3. Technique et Documentation Ed.—Paris.

La technique la plus ancienne pour extraire les protéines de lactosérum consiste à les insolubiliser par un traitement thermique dénaturant à un pH proche de leur point isoélectrique. L'inconvénient évident d'une telle technique est de dénaturer les protéines. D'autres procédés ont donc été proposés pour isoler les protéines par précipitation et/ou complexation. On peut citer par exemple l'adsorption des protéines sur échangeur d'ions ou l'addition de réactifs chimiques en vue de la complexation et de la précipitation des protéines. Ces diverses techniques sont sensiblement restées au niveau du laboratoire. On a par ailleurs suggéré des procédés chromatographiques d'échangeur d'ions. Voir MIRABEL, B. (1978)—Nouveau procédé d'extraction des protéines du lactosérum. Ann. de la Nutrition et de l'Alim., *32*(2—3), 243—253. La filtration sur gel a été également appliquée au lactosérum. Un tel procédé de filtration présente un certain nombre d'inconvénients. Il exige notamment une préconcentration des protéines qui ne devra pas être dénaturante au risque d'altérer la résolution entre les différentes fractions. Ces dernières doivent ensuite être elles mêmes concentrées et séchées. Les difficultés de mise en oeuvre expliquent aussi pourquoi cette technique est restée très limitée.

L'ultrafiltration sur membrane, étant donné les progrès réalisés tant sur les appareillages que dans la compréhension des phénomènes observés, s'est largement répandue dans l'industrie laitière pour le traitement du lait [MAUBOIS J. L., MOCQUOT, G. (1971 )—Préparation de fromage à partir de "pré-fromage liquide" obtenu par ultrafiltration du lait. Le lait, *51* 508, 495—533], ainsi que pour celui du lactosérum (FENTONMAY R. I., HILL C. G. et AMUNDSON, C. H. (1971)—Use of ultrafiltration-reverse osmosis systems for the concentration and fractionation of whey, J. Food Sc, *36*, 14). Lors du passage du lactosérum sur la membrane d'ultrafiltration, l'eau, les minéraux solubles, le lactose, les composés azotés de faible poids moléculaire (peptides, acides aminés libres) et les vitamines hydrosolubles traversent la membrane sous forme d'ultrafiltrat ou perméat; par contre, les protéines et les constituants associés (calcium, phosphore) les globules gras et les éléments lipophiles sont retenus et se concentrent au fur et à mesure de l'élimination de la phase aqueuse: ils constituent le rétentat ou concentré protéique. L'obtention de concentrés proteiques de pureté élevée nécessite la mise en oeuvre d'une ultrafiltration ainsi que d'une diafiltration. Dans la diafiltration, on effectue une addition d'eau ou de solution aqueuse contenant des sels, en continu ou en discontinu, dans le rétentat d'ultrafiltration. On élimine simultanément ou successivement une quantité équivalente de perméat. Une telle opération a pour conséquence d'appauvrir le rétentat en éléments filtrables. Outre des qualités nutritionnelles certaines, les protéines de lactosérum, sous forme concentrée, possèdent des propriétés fonctionnelles intéressantes. De nombreuses revues bibliographiques existent sur ce sujet: voir par exemple KINSELLA, J. E. (1976)—Functional properties of proteins in foods; a survey. Critical reviews in food science and nutrition.

L'invention a pour objet une utilisation originale des protéines de lactosérum, en particulier en vue de la nutrition humaine. Les progrès rapides de la médecine et de la chirurgie permettent la survie de nombreux malades chez lesquels la nutrition, la réadaptation à une alimentation normale deviennent un problème important (progrès des techniques chirurgicales mais aussi des techniques de réanimation). A chaque stade de la réanimation, de la thérapeutique métabolique à l'alimentation normale, le mélange nutritif offert doit être adapté étroitement au besoin du malade.

Les nouvelles techniques d'alimentation telles que l'alimentation entérale à faible débit continu (voir LEVY E. (1976)—Alimentation entérale continue. Principe-Technique. Indications, Résultats. 64, (4), 235—256, permettent l'utilisation optimale des nutriments par leur acheminement direct, en l'état, au niveau de leur lieu d'utilisation ou d'action. Ainsi, après avoir déterminé la fonction digestive déficiente, il s'agit de la suppléer et éventuellement de stimuler son retour à une activité quasi-normale. La fabrication industrielle de ces produits de substitution consiste en fait à reproduire in vitro la fonction déficiente qui peut aller de l'impossibilité d'ingérer, de mastiquer, à certaines carences ou déficiences enzymatiques.

On sait par ailleurs que la protéolyse réelle des protéines solubles du lait intervient au niveau intestinal sous l'action des protéases pancréatiques. Voir les références de BLANC B. (1976—Digestibility of Proteins. Int. Dairy Federation, Commission F, Final Report, Doc. 57 GRAY, G. M., COOPER, H. L. (1971)—Protein digestion and absorption. Gastroenterology, *61*, 535. On cherche donc le plus possible à l'heure actuelle à mettre au point des hydrolysats enzymatiques qui sont capables d'être aisément absorbés par l'organisme humain.

L'invention concerne l'obtention à partir de protéines de lactosérums d'un hydrolysat protéique capable d'être directement assimilable par la muqueuse intestinale et pouvant avoir une action de stimulation au niveau des systèmes enzymatiques déficients.

On connaît enfin un certain nombre de procédés pour l'hydrolyse des protéines, par exemple des protéines de lait, L'hydrolyse acide conduit effectivement à l'obtention de solutions d'acides aminés libres. L'hydrolyse acide détruit certains acides aminés. L'hydrolyse alcaline préserve le tyrptophane mais entraîne une insolubilisation qui diminue fortement la valeur nutritionnelle des concentrés protéiques initiaux.

La protéolyse enzymatique est connue et pratiquée depuis fort longtemps, à des fins analytiques ou à des fins alimentaires, le but principal étant de solubiliser les protéines. La littérature fait largement état de nombreuses utilisations alimentaires d'hydrolysats de protéines de soja (Voir ARAI, S. NOGUCHI M. KUROSAWA, S. KATO, H. et FUJIMAKI, M. (1970) Applying proteolytic enzymes on soybean, 6-Deodorization effect, des protéines de poissons: voir HEVIA, P. WHITAKER, J. R. et OLCOTT, H. S. (1976)—Solubilization of a fish protein concentrate with proteolytic enzymes. J. Agric. Food Chem., Vol 24 (2) 383—385, ou de colza, par action de protéase animale, microbienne ou végétale.

L'application de ces techniques aux protéines laitières au niveau industriel reste cependant très limitée. La protéolyse enzymatique ne présente pas les inconvénients des méthodes chimiques. Les conditions d'hydrolyse sont modérées et préservent ainsi la qualité nutritionnelle des produits.

En général, l'hydrolyse conduit à des peptides ayant un goût amer prononcé. Ce caractère limite l'utilisation de ces hydrolysats en alimentation humaine. L'intensité de l'amertume d'un hydrolysat dépend principalement de la nature du substrat protéique et de la spécificité des enzymes. Pour éliminer l'armeture, on a suggéré de faire agir des exopeptidases. Voir par exemple ARAI, S. YAMASHITA, M., KATO, H. FUJIMAKI, M. (1970)—Agric. Biol. Chem.; 34, 729, ainsi que CLEGG, K. M. SMITH, G. et WALKER, A. L. (1974)—Production of an enzymatic hydrolysate of casein on a kilogram scale. J. Food Technol. 9, 425—431. On a également proposé de modifier les peptides par addition d'acides glutamiques avant réaction plastéique. Il est également possible de recourir à une élimination des acides aminés hydrophobes.

Cependant, toutes ces techniques connues ne sont pas satisfaisantes et ne peuvent pas répondre aux besoins de l'invention. En effet, une solubilisation poussée, causée par la mise en oeuvre d'exopeptidase, augmente le taux d'acides aminés libres et plus spécialement l'arginine, la lysine, la tyrosine, la valine, la phénylalanine, la méthionine et la leucine, ce qui aurait pour effet direct d'encombrer les systèmes de transport, au niveau de la barrière intestinale des acides aminés libres et donc d'entraîner une moindre efficacité nutritionnelle des hydrolysats. Par ailleurs, la qualité intrinsèque des hydrolysats est modifiée car l'équilibre en acides aminés est lui-même changé, ce qui nécessite une supplémentation en acides aminés libres.

A titre de document illustrant la technique antérieure, on peut citer le brevet US 2. 180.637. Ce brevet décrit un procédé pour produire des acides aminés purifiés à partir de certaines fractions protéiques, la caséine du lait étant préférée. La lactalbumine est également mentionnée comme source de protéines appropriée. Le procdé peut consister en une hydrolyse enzymatique en milieu basique et fournit un mélange d'acides aminés purs non dénaturés. Parmi les enzymes utilisables sont citées les enzymes pancréatiques. Une telle hydrolyse conduit essentiellement à des acides aminés libres à côté d'une faible quantité de polypeptides, ainsi qu'il est explicitement mentionné dans la description du brevet US 2.180.637. Le procédé décrit dans ce brevet a pour but de fournir des hydrolysats protéiques contenant une proportion maximale d'acides aminés libres grâce à une hydrolyse totale qui doit être réalisée avec un temps de contact prolongé entre l'enzyme et le substrat. Le produit ainsi obtenu n'est donc pas forcément exempt d'enzymes. De plus, la composition du produit obtenu par le brevet US 2.180.637, en raison de sa composition en acides aminés libres, ne convient pas pour certains domaines d'application tels que pour la réanimation et la nutrition thérapeutique humaine. Des études récentes ont en effet révélé qu'il convenait d'utiliser des mélanges de peptides plutôt que des acides aminés libres (voir par exemple D. B. A. SILK in Peitide Transport and Hydrolysis CIBA Foundation Symposium 50 1977 Elsevier Exurpta Medica North. Holland). L'administration de produits essentiellement constitués par des acides aminés libres est donc déconseillée en raison des inconvénients que cela peut présenter lors de l'application clinique, du fait que les acides aminés libres ne sont pas assimilables immédiatement et sans danger par l'organisme humain (voir par exemple D. M. MATTHEWS in Physiological Reviews Vol. 55 n° 4 October 1975 "Intestinal Absorption of Peptides"). Les études modernes indiquent qu'il faut tenir compte de l'osmolalité des produits et on sait que l'osmolalité des produits peptidiques est très inférieure à celle des produits correspondants constitués d'acides aminés libres. Plus l'osmolalité est basse, plus la faculté d'assimilation immédiate par l'organisme humain est grande. Il existe donc un besoin pour trouver des produits capables d'être assimilés immédiatement et sans danger par l'organisme humain, et pouvant convenir, par exemple, en nutrition thérapeutique et en réanimation.

Le brevet US-A 3950547 concerne des compositions diététiques, consommables sous forme d'émulsions aqueuses, comprenant des peptides et/ou des acides aminés, des lipides et des hydrates de carbone, ainsi que, de préférence, de l'amylose. Le mélange de peptides est constitué de peptides ayant un poids moléculaire entre 400 et 1000, et au plus de 2000, et leur maximum de distribution se situe entre 4 et 8 résidus d'acides aminés. Le mélange est obtenu de manière traditionnelle par hydrolyse enzymatique ou chimique de farine de poisson, de protéines de graines oléagineuses, de protéines de feuilles, de protéines unicellulaires ou de déchêts animaux d'abattoirs et de sang, par exemple par hydrolyse selon le procédé du brevet US-A 2180637. En plus des peptides, l'hydrolysat contient aussi environ 10-15% en poids d'acides aminés libres, lysine, arginine, tyrosine, phénylalanine et leucine.

Ce brevet US-A 3950547 ne divulgue pas les moyens d'obtenir un mélange peptidique par hydrolyse enzymatique de protéines de lactosérum, dont la composition et la répartition de taille des peptides sont bien déterminés et correspondent au produit existant dans l'organisme humain après digestion desdites protéines de lactosérum.

A la connaissance du demandeur, il n'a jamais été proposé de procédé pour l'obtention d'un hydrolysat enzymatique total utilisant les protéines du lactosérum et pouvant servir directement dans les besoins spécifiques de l'alimentation humaine.

Sur le plan technologique, l'hydrolyse enzymatique fait le plus souvent appel à un système de réacteur discontinu. L'enzyme est ajoutée à la solution protéique à traiter. Après un temps de séjour plus ou moins long, dans des conditions favorables à l'activité enzymatique et à l'attaque du substrat, le pH est modifé et un traitement thermique léger inactive l'enzyme. Une centrifugation peut être faite pour éliminer la fraction insoluble non digérée. Mais, selon cette technique de réaction d'hydrolyse enzymatique discontinue, il est difficile d'utiliser un rapport enzyme/substrat élevé. Or, on connaît, voir ROBINS, R. C. (1978)—Effect of ratio of enzymes to substrates on amino acid patterns released from proteins un vitro. Internat. J. Vit. Nutr. Res., 48, 44—52, l'influence décisive du rapport enzyme substrat sur la nature des acides aminés libres et les peptides libérés lors de la protéolyse. Avec un procédé discontinu, il faut détruire les enzymes en fin d'hydrolyse lorsque celles-ci sont en excès, ce qui serait indispensable avec les rapports élevés précités.

On a également proposé d'utiliser des réacteurs à enzymes fixés. Cependant, ceux-ci présentent de notables inconvénients sur le plan pratique. En effet, les conditions optimales d'activité des enzymes, en particulier les conditions de pH, se déplacent au cours du temps de sorte que le fonctionnement du réacteur ne donne pas satisfaction en permanence. On constate également des problèmes bactériologiques, le colmatage des lits de fixation ainsi que l'adsorption des protéines sur le support dans le cas du traitement du lactosérum. En outre, la réaction enzymatique a tendance à s'inhiber au cours du temps en raison de la formation de complexe enzymefragment protéique. L'inhibition peut être également due à la nature du support. Il est par ailleurs très difficile d'utiliser des systémes multi-enzymes en raison des phénomènes de compétition des enzymes vis-à-vis du support et de la stabilité différente des enzymes au cours du temps.

L'invention a mis a profit les moyens déjà connus dans certaines autres applications et qui consistent à faire appel à des réacteurs enzymatiques à membranes. On peut se référer par exemple à l'article de CHEFTEL, C. (1972) (Solubilisation enzymatique continue du concentré protéique de poisson. Essai de recyclage des enzymes. Ann. Technol. Agric.; 21 (3) 423—433, qui décrit un réacteur à membranes, appliqué à la protéolyse de concentré protéique de poisson. La membrane d'ultrafiltration permet de retenir l'enzyme en solution dans le réacteur ainsi que le substrat protéique. Seuls les produits d'hydrolyse, les peptides, sont éliminés au fur et à mesure de leur formation. Cependant, dans la pratique, la mise en oeuvre d'un tel réacteur n'est pas aisée comme le souligne CHEFTEL. Le substrat doit être complètement solubilisable par l'enzyme et la solution protéique doit présenter une qualité bactériologique irréprochable.

Tel est brièvement rappelé ci-dessus l'état de la technique dans le domaine de la valorisation des protéines du lactosérum, en particulier par hydrolyse enzymatique. On peut dire en résumé qu'aucun des documents connus du demandeur n'a proposé de réaliser un hydrolysat enzymatique total de protéine de lactosérum.

Sous sa forme la plus générale, l'invention a pour objet hydrolysat enzymatique total de protéines de lactosérum contenant des peptides caractérisé en ce qu'il ne contient pas de protéines résiduelles, en ce qu'au moins 50% des peptides possèdent 2 à 5 acides aminés et en ce que la proportion d'acide aminé libre est inférieure à 15%.

Selon un mode préféré de réalisation, les hydrolysats mentionnés ci-dessus contiennent 70 à 90% de l'azote présent sous forme de peptides ayant un nombre d'acides aminés inférieur à 10.

L'invention concerne également un procédé pour l'obtention de tels hydrolysats à partir de protéines de lactosérum dans lequel on soumet un concentré protéique de lactosérum à une hydrolyse enzymatique cntinue avec une enzyme proléolytique capable de reconstituer la digestion protéique qui prend place in vivo dans l'organisme humain, en introduisant ce concentré avec l'enzyme dans une zone de réaction, on soutire en continu le produit de réaction en l'amenant de la zone de réaction dans une zone d'ultrafiltration, de laquelle on soutire également en continu un perméat qui représente l'hydrolysat peptique constituant le produit recherché, la réaction d'hydrolyse étant poursuivie jusqu'à ce que le produit ne contienne plus de protéines résiduelles, c'est-à-dire ne présente aucun azote précipitable par l'acide trichloracétique à 12%, et jusqu'à obtention d'un hydrolysat peptidique dont au moins 50% des peptides contiennent 2 à 5 acides aminés, un recyclage pouvant être prévu de la zone d'ultrafiltration jusqu'à la zone de réaction. Ladite enzyme 257 de préférence la pancréatine, l'hydrolyse étant conduite jusqu'à ce que le produit ne contienne plus de protéines résiduelles c'est-à-dire ne présente aucun azote précipitable par l'acide trichloracétique à 12%, après quoi on récupère l'hydrolysat obtenu qui représente l'hydrolysat enzymatique total recherché.

Sous une forme préférée, l'invention concerne un procédé dans lequel on effectue d'abord l'ultrafiltration

EP 0 022 019 B2

du lactosérum, ledit procédé étant caractérisé en ce qu'on met en contact le rétentat d'ultrafiltration avec une enzyme protéolytique capable de reconstituer la digestion protéique qui prend place in vivo dans l'organisme humain, ladite enzyme étant de préférence la pancréatine, l'hydrolyse étant conduite jusqu'à ce que le produit ne contienne plus de protéines résiduelles c'est-à-dire ne présente aucun azote précipitable par l'acide trichloracétique à 12%, près quoi on récupère l'hydrolysat obtenu qui représente l'hydrolysat enzymatique total recherché.

Ainsi qu'on l'a mentionné précédemment, un tel procédé est avantageusement mis en oeuvre dans un dispositif qui combine un équipement d'ultrafiltration à un réacteur enzymatique à membrane, permettant ainsi une réalisation continue. Grâce à une telle combinaison et au choix des paramètres de la réaction qui sont adaptés au traitement des protéines du lactosérum l'invention permet de réaliser une hydrolyse enzymatique totale desdites protéines. Jusqu'à présent, les hydrolyses réalisées n'étaient que partielles, et par le fait même les produits obtenus ne correspondaient pas au besoin de la présente invention.

La quasi totalité des peptides obtenus est constituée de 2 à 5 acides aminés, ce qui permet au produit de l'invention de répondre aux besoins nutritionnels spécifiques. Les acides aminés libres représentent 10 à 15% de la fraction azotée, en général. Les acides aminés basiques (arginine et lysine) aromatiques (tryptophane, phénylalanine, tyrosine) et la leucine constituent l'essentiel de la fraction des acides aminés libres. Les exemples ci-après mettront en évidence les compositions de certaines catégories d'hydrolysats données à titre illustratif.

La similitude de composition en acides aminés de la fraction floculante et de la fraction surnageante obtenue lors de la concentration par évaporation à 100g par litre de l'hydrolysat sortant du réacteur indique une égale répartition des caractères hydrophobe et hydrophile entre les différentes classes de peptides. L'opération de concentration peut être poursuivie jusqu'à une teneur en substance sèche élevée, pouvant aller jusqu'à 25% avant séchage par atomisation.

Les hydrolysats obtenus sont issus de protéines laitières reconnues comme ayant une excellente valeur biologique. Ils répondent donc aux besoins nutritionnels des malades souffrant d'insuffisance pancréatique ou de malades métaboliques. L'invention trouve donc une application directe dans des aliments diététiques parfaitement assimilables par l'organisme humain puisque capables de passer directement la barrière intestinale et d'être ainsi commodément digérés et assimilés. Sous l'un de ses aspects, l'invention concerne donc un aliment de réanimation contenant, à titre de fraction active, un produit de l'invention à base d'hydrolysat enzymatique total des protéines de lactosérum, en combinaison éventuelle avec un véhicule neutre convenable pour l'alimentation orale ou entérale.

A titre de matière de départ pour le procédé de l'invention, on peut utiliser n'importe quel lactosérum. On peut par exemple utiliser des lactosérums d'origine industrielle, en particulier des lactosérums mixtes résultant du mélange de lactosérums de diverses productions fromagères. On peut mettre en oeuvre un lactosérum directement préparé à partir de lait cru ou thermisé, par précipitation lactique ou présure. Mais, en variante, on peut également utiliser un lactosérum reconstitué. Le lactosérum d'origine, après écrémage, peut être thermisé, par exemple à une température de l'ordre de 58—60°C. Il peut ensuite être concentré par évaporation. Le préconcentré est éventuellement dilué pour éviter la cristallisation excessive du lactose et conservé à une température de l'ordre de 4°C. Il est en effet commode d'utiliser des concentrés protéiques de lactosérum reconstitués à partir de poudre.

Le procédé de l'invention est applicable aux protéines du lactosérum quelle que soit leur technique d'obtention. Ainsi, on peut isoler les protéines de lactosérum par tout moyen connu avant de les soumettre à l'hydrolyse enzymatique. On peut par exemple faire coaguler ces protéines par la chaleur. On donne la préférence à une obtention des protéines par ultrafiltration du lactosérum, car cette opération n'entraîne aucune dénaturation. Pour ces raisons diverses, économiques par exemple, le procédé de l'invention est cependent applicable avec succès aux protéines précipitées par chauffage du lactosérum.

Le lactosérum ou préconcentré est avantageusement purifié par ultrafiltration et par diafiltration. Les différents rétentats sont ensuite séchés, par exemple par atomisation.

Lors de l'ultrafiltration, on utilise les techniques connues de l'homme de l'art, telles qu'elles sont notamment illustrées par les références bibliographiques citées au début de la description. L'obtention d'un concentré protéique de pureté élevée s'effectue en général en deux étapes: ultrafiltration simple suivie d'une diafiltration. Les membranes d'ultrafiltration sont conventionnelles: ce sont celles qui sont connues pour retenir toutes les protéines solubles du lactosérum. Leur pouvoir de coupure est en général compris entre 500 et 50000. La nature de ces membranes est quelconque. Il peut s'agir aussi bien de membranes inorganiques qu'organiques.

A titre de membranes organiques, on peut utiliser des membranes en polyoléfine, polyacrylonitrile, chlorure de polyvinyle, acétate de cellulose, polycarbonate et autres matières similaires. A titre de membranes inorganiques, on peut utiliser n'importe quel substrat poreux répondant aux définitions ci-dessus. Toutes ces notions sont bien connues de l'homme de l'art dans le domaine de l'ultrafiltration du lait ou du lactosérum et n'ont donc

6

pas besoin d'être explicitées davantage.

Les meilleurs résultats ont été obtenus avec les membranes disponibles sur le marché sous la dénomination "Nuclepore". De telles membranes sont décrites dans la brochure intitulée "Basics of filtration and separation" de H. W. BALLEW et Nuclepore Corporation (1978). Ces membranes sont constituées d'un film de polycarbonate irradié par bombardement ionique. Les pores sont des trous cylindriques bien définis dont le diamètre est précis à plus ou moins 15%. Ces membranes de microfiltration ont non seulement la faculté de supprimer les produits insolubles protéiques, lipidiques ou lipoprotéiques mais également de retenir les microorganismes et d'exercer une fonction supplémentaire de stérilisation du produit.

Dans la pratique, afin de réaliser des conditions optimales d'ultrafiltration du lactosérum, on ajustera le pH à une valeur généralement comprise entre 6 et 7 pour tenir compte de l'origine du lactosérum (coagulation acide ou présure). La modification du pH peut améliorer de façon sensible les performances des appareillages. Cette amélioration est accrue lorsque l'ajustement du pH intervient après préchauffage du lactosérum à 80°C pendant 15″: voir HAYES, J. F. DUNKERLEY, J. A. MULLER, L. L. GRIFFIN, A. T. (1974) Studies on whey processing by ultrafiltration. II. Improving permeation rates by preventing fouling. The Australian J. Of Dairy Tech., *37*, (3), 132—140. En outre, l'élimination de certains minéraux améliore les débits de perméation. De même, le passage du lactoserum sur une membrane d'ultrafiltration dont le seuil de coupure se situe au-dessus des poids moléculaires des protéines natives élimine les microorganismes, les fines de caséine, les protéines solubles sous forme d'aggrégats. Cette préfiltration de lactosérum augmente les débits de perméation: voir les travaux de LEE, D. N. & MERSON, R. L. ( 1976) Chemical treatments of cottage cheese whey to reduce fouling of ultrafiltration membrane. J. of Food Sc., *41*, 778—786. LEE, D. N. & MERSON, R. L. (1976)—Prefiltration of cottage cheese whey to reduce fouling of ultrafiltration membranes. J, of Food Sc., *41*, 403—410. Ces mêmes auteurs ont constaté que l'addition d'un sel inorganique tel que du chlorure de calcium ou de chlorure de sodium améliore le débit d'ultrafiltration. Ce traitement peut être envisagé lors de l'opération de diafiltration en vue d'obtenir des concentrés protéiques très purs dans les conditions optimales.

Conformément à une forme de mise en oeuvre avantageuse de l'invention on combine l'étape d'ultrafiltration du lactosérum à une étape d'hydrolyse enzymatique du rétentat de ladite ultrafiltration. Quel que soit le mode d'obtention des protéines de lactosérum, l'hydrolyse enzymatique est réalisée en continu en introduisant le concentré protéique dans une zone de réaction en vue d'une mise en contact intime avec l'enzyme, on soutire en continu le produit de réaction en l'amenant de la zone de réaction dans une zone d'ultrafiltration, de laquelle on soutire également en continu un perméat qui constitue l'hydrolysat peptidique constituant le product recherché selon l'invention, la réaction étant poursuivie jusqu'à ce que le perméat soit un hydrolysat enzymatique total, un recyclage pouvant être prévu de l'ultrafiltration jusqu'à la zone de réaction.

Pour des raisons liées à la fois au bon fonctionnement des réacteurs et à la destination des produits finals, l'élimination des composés non protéiques du lactosérum doit être la plus élevée possible. Il convient donc que, dans le rétentat d'ultrafiltration précédemment mentionné, le repport protéine/matière sèche soit le plus élevé possible. C'est notamment pour cette raison qu'une diafiltration a été proposée après une d'ultrafiltration. Dans le cas cependant où la présence une faible quantité de lactose résiduel pourrait présenter quelques inconvénients sur la composition du produit final, l'hydrolyse de ce sucre peut être réalisée. Des essais ont montré que l'hydrolyse enzymatique préalable des concentrés de protéine de lactosérum n'avait acune conséquence néfeste pour la suite du procédé d'hydrolyse protéique.

On donnera maintenant des indications sur les paramètres de la réaction enzymatique, lesquels présentent une grande importance pour obtention d'un hydrolysat enzymatique total.

En premier lieu, le pH de la zone de réaction doit être ajusté entre 7 et 9 compte-tenu de la nature des enzymes utilisées, lesquelles doivent être capables de digérer totalement les protéines de lactosérum dans les conditions de la digestion d'un organisme humain. A titre d'enzymes, on met donc en oeuvre de préférence de la pancréatine qui est un mélange complexe contenant de la trypsine, de la chymotripsine et d'autres enzymes protéolytiques. Dans la pratique, on utilisera un extrait pancréatique naturel disponible sur le marché et aisément accessible. Toutefois, si besoin est, on peut également mettre en oeuvre des enzymes résultant d'un mélange synthétique dont la composition se rapproche de la pancréatine. De tels mélanges contiendront donc essentiellement de la trypsine et de la chymotripsine, le cas échéant avec d'autres enzymes secondaires présentes dans l'extrait pancréatique naturel. On a trouvé selon l'invention qu'au pH compris entre 7 et 9, et de préférence entre 7 et 8,5, la pancréatine et autres enzymes analogues répondant aux besoins de l'invention présentaient une stabilité maximale.

Il convient d'observer également des conditions assez strictes de température dans la zone de réaction enzymatique. On a constaté en effet que l'activité des enzymes était davantage influencée par la température que par le pH. Des essais ont montré notamment selon l'invention qu'avec la trypsine la température maximale dans la zone de réaction ne devait pas être supérieure à 54°C, et avec la chymotripsine cette température ne devait pas dépasser 45°C. Dans la pratique, lorsqu'on utilise de la pancréatine, on adoptera donc un compromis

7

tenant compte à la fois des conditions optimales de la protéolyse intestinale (température entre 37 et 40°C) et du fait qu'une température plus élevée est moins favorable du développement des germes et permet d'obtenir des débits d'ultrafiltration supérieurs. Pour les besoins de l'invention des températures de l'ordre de 40°C se sont donc avérées convenables.

Bien entendu, les paramètres de la réaction, pH et température, sont liés. Ainsi, des gammes de pH de 7,5 à 8,5 pour des températures de l'ordre de 40 à 45°C se sont avérées convenables.

Un autre paramètre important dans la zone de réaction est le rapport entre la concentration en enzymes et la concentration en protéines. Ce rapport doit être élevé, comme cela est le cas au cours de la digestion protéique in vivo. Dans le cas de la pancréatine, des rapports pancréatine/substrat de l'ordre de 8 à 15%, en particulier de l'ordre de 12%, ont donné des résultats convenables.

Tous les paramètres ci-dessus sont choisis de manière que la totalité des protéines soit protéolysée. Ceci évite une augmentation de leur concentration en fonction du temps dans le réacteur. Par ailleurs, la solution protéique alimentant le réacteur d'hydrolyse enzymatique ne doit pas comporter de composants non protéiques susceptibles d'être retenus pas la membrane. L'enzyme doit également être retenue effectivement par la membrane. Enfin, s'agissant de produits destinés à l'alimentation, les conditions de fonctionnement ne doivent pas être favorables à un développement microbien.

Pour réaliser une hydrolyse enzymatique optimale, il convient également de choisir avec soin la membrane d'ultrafiltration à utiliser en relation avec le réacteur enzymatique. Les membranes utilisées sont de type quelconque, organique ou inorganique. Elles peuvent se présenter sous forme de modules ainsi qu'il est connu dans ce genre d'appareillage. Une organisation des membranes ayant donné de bons résultats est celle des modules à fibres creuses. A titre particulier, on peut utiliser les membranes de la Société AMICON disponibles sur le marché sous la dénomination H10 P 5 (seuil de coupure 5000) et H10P10 (seuil de coupure 10.000) ainsi que les membranes de la Société ROMICON disponibles sur le marché sous la dénomination PM 2, (seuil de coupure 2000) ou PM 50 (seuil de coupure 50.000).

Il est important de choisir une membrane qui, en fonctionnement, puisse répondre aux besoins de la présente invention et en particulier retenir efficacement l'enzyme et présenter des performances satisfaisantes, en particulier pour la durée. En effet, la matière première traitée, c'est-à-dire la solution protéique issue du lactosérum, peut posséder des composants qui nuisent au bon fonctionnement de la membrane. Au cours du processus d'obtention du lactosérum, une fraction protéique insolubilisée, peut être présente en raison du traitement thermique habituel, 72°C—15″. C'est pourquoi il est préférable d'éliminer au préalable cette fraction protéique par centrifugation à vitesse élevée. Le lactosérum peut également contenir une fraction essentiellement constituée de lipides et de complexes lipoprotéiques. Ces composants, bien que de taille moléculaire élevée, ne sont pas centrifugeables. Leur accumulation au droit de la membrane utilisée en relation avec le réacteur enzymatique peut limiter le fonctionnement du réacteur.

Selon une forme préférée de réalisation de l'invention, on met en oeuvre des membranes dont le profil des pores est bien déterminé.

Sur un autre plan, la destination des hydrolysats protéiques selon l'invention exige que les produits entrant dans le réacteur soient de bonne qualité hygiénique bactériologique. L'addition généralisée de nitrate de potassium au lait utilisé en fabrication de fromages pressés peut conduire soit à rejeter le lactosérum issu de cette transformation soit à réaliser un traitement supplémentaire de déminéralisation par électrodialyse ou sur résine échangeuse d'ions, si le taux résiduel de nitrate est jugé inacceptable.

La présence de peroxyde d'hydrogène, antiseptique largement employé dans la stabilisation bactériologique du lactosérum, ne saurait non plus être acceptée.

Compte-tenu de son processus de fabrication et de sa composition (faible teneur en matières grasses et population bactérienne réduite) le lactosérum issu de la fabrication de caséine à l'aide d'acides minéraux est une matière première préférée pour le procédé de l'invention.

Ainsi qu'un l'a mentionné ci-dessus, il est important de maintenir dans la zone de réaction un pH basique de l'ordre de 7 à 9 en vue de permettre la digestibilité totale du concentré protéique. A cet effet, on introduit en continu ou en discontinu dans la zone de réaction un composé basique qui peut être l'hydroxyde ou le carbonate de sodium, l'hydroxyde ou le carbonate de potassium, l'hydroxyde de calcium, l'hydroxyde d'ammonium ou un mélange de ces produits. Le choix d'un composé basique particulier dépendra de la destination du produit final, la teneur en ions sodium, potassium, calcium ou ammonium dans ledit produit étant alors le critère déterminant de choix.

L'homme de l'art disposera donc d'un certain nombre de paramètres pour obtenir les meilleurs résultats et réaliser l'hydrolyse enzymatiques totale des protéines de lactosérum. On notera également que les valeurs particulières à choisir peuvent dépendre de la taille du réacteur enzymatique ainsi que la nature des équipements utilisés.

Il conviendra de conduire l'hydrolyse des protéines du lactosérum jusqu'à l'obtention d'hydrolysats enzy-

matiques ne contenant plus de protéines décelables. On met alors l'hydrolysat en contact avec la membrane du module d'ultrafiltration combiné au réacteur enzymatique.

Le procédé de l'invention peut être mis en oeuvre en deux étapes séparées: une première étape consistant en l'hydrolyse enzymatique et une deuxième étape consistant en l'ultrafiltration. Les équipements pour la mise en oeuvre de chacune de ces opérations peuvent être séparés ou intégrés. Mais, en variante, le procédé peut également être exécuté en continu, les deux étapes ci-dessus étant réalisées dans un appareillage unique. Pendant les premiers instants de fonctionnement, par exemple pendant 1 heure environ, le perméat (liquide passant à travers la membrane) est recyclé dans la zone d'hydrolyse afin d'obtenir le taux d'hydrolyse désiré des protéines de lactosérum. L'hydrolyse totale étant atteinte, on introduit dans le réacteur le concentré protéique à traiter à un debit identique à celui du perméat. Dans des conditions particulières impliquant une température de 40°C environ, un pH maintenu à 8,0 dans la zone de réaction, une teneur en protéines d'environ 80 g par litre de la solution alimentant le réacteur et un rapport pancréatine/substrat de 12% environ, la durée totale de fonctionnement en continu était de l'ordre de 7 à 8 heures.

On notera enfin qu'un traitement thermique préalable des solutions concentrées de protéines de lactosérum n'améliorent pas leur digestibilité globale.

Parmi les protéines présentes dans le concentré soumis à protéolyse, on a trouvé selon l'invention que la sérumalbumine était celle qui était la plus difficilement hydrolysée par la pancréatine.

Les produits de l'invention peuvent recevoir de nombreuses applications dans le domaine alimentaire et thérapeutique. Les produits de l'invention sont de nature à déclencher, par action sur les récepteurs intestinaux, la sécrétion d'hormones pancréatiques internes (insuline et glucagon, par exemple) et de déclencher la sécrétion d'hormones gastro-intestinales (gastrine, polypeptide inhibiteur gastrique, par exemple).

Les produits de l'invention peuvent être utilisés en vue de la nutrition thérapeutique ou à titre de médicament en réanimation: ils permettent d'obtenir une amélioration de la trophicité de l'intestin réséqué permettant de retrouver après un certain temps une autonomie nutritionnelle et ainsi de cesser de nourrir le malade par sonde. Plus généralement les produits de l'invention peuvent être utilisés en vue de la nutrition thérapeutique ou à titre de médicament dans le traitement des infections du type digestif comme par exemple les gastrites, les ulcères gastro-duodéniques, les iléites, les intestins réséqués et les colites.

L'invention concerne aussi des compositions pharmaceutiques contenant un produit de l'invention en mélange avec les excipients usuels.

Compte tenu de la forme physique du nouveau produit (poudre soluble en milieu aqueux), la forme de présentation ne soulève pas de difficulté. Les nouveaux produits peuvent être ingérés ou administrés en tant que tels par voie entérale, par exemple en mélange avec l'aliment habituel. Ils peuvent également être présentés sous forme de compositions avec des excipients usuels, par exemple convenant à l'administration orale. Des compositions appropriées aux fins de l'invention peuvent ainsi être présentées sous forme de comprimés ou de gélules, avec des excipients connus tels que le talc, le stéarate de magnésium, la silice finement divisée, et tout autre véhicule analogue connu de l'homme de l'art.

L'invention sera maintenant illustrée à l'aide d'exemples. Bien entendu, ceux-ci ne sont nullement limitatifs. Dans les exemples ci-après, il a été fait usage d'un dispositif décrit en référence aux dessins annexés sur lesquels:

Fig. 1 représente une vue schématique générale du dispositif.

Fig. 2 est un schéma représentant le réacteur enzymatique à membrane utilisé dans le dispositif de la figure 1.

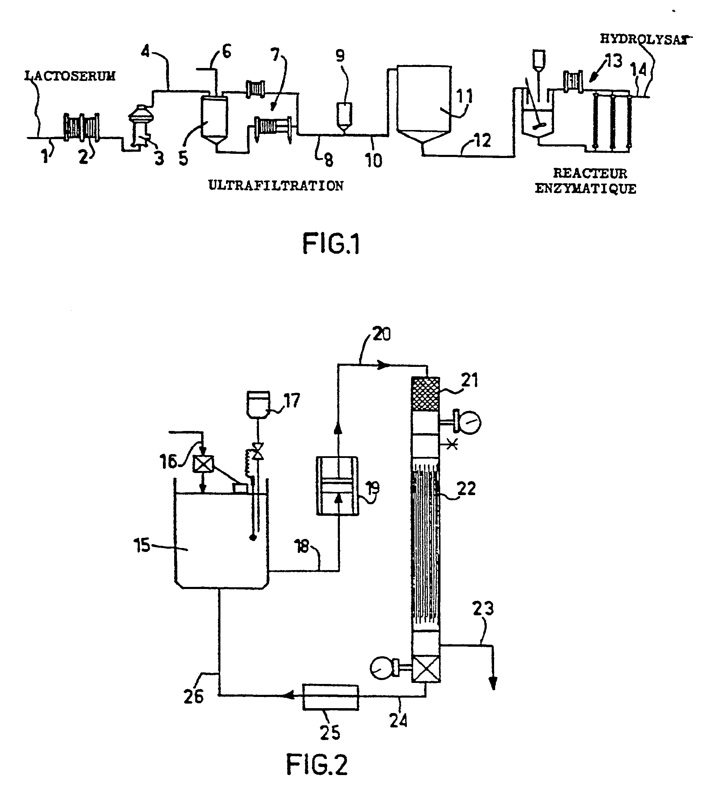

Ainsi que le représente la figure 1, le dispositif comprend: une installation d'ultrafiltration et un réacteur enzymatique. Le lactosérum est introduit par la canalisation 1 pour traverser d'abord un échangeur de température 2 et un séparateur centrifuge 3 avant d'être introduit dans le module d'ultrafiltration proprement dit. Le lactosérum réchauffé et débarrassé des insolubles centrifugeables est introduit par la canalisation 4 dans un réservoir 5, on même temps que de l'eau alimentée par la canalisation 6. L'eau est utilisée en continu en vue de la diafiltration. Le module d'ultrafiltration proprement dit est représenté globalement par la référence 7; on récupère le rétentat dans la canalisation 8. On a schématisé en 9 les équipements nécessaires à l'ajustement du pH du rétentat ainsi qu'à sa concentration. Une fois ces opérations effectuées, le rétentat passé par la canalisation 10 et est introduit dans une cuve-tampon 11 de laquelle il est prélevé pour être hydrolysé dans le réacteur enzymatique. A cet effet, le lactosérum passe par la canalisation 12 piquée sur la cuve 11 pour être amené au réacteur enzymatique désigné par la référence générale 13. L'hydrolysat qui constitue le produit final recherché est récupéré à la canalisation 14.

Pour des essais spécifiques, le lactosérum ou le préconcentré a été ultrafiltre sur un module DDS d'une surface de 9 m², équipé de membranes BR 6 P également mises sur le marché par la Société DE DANSKE SUKKER FABRIK, l'opération d'ultrafiltration étant réalisée en discontinu à la température de 50°C. Les différents rétentats obtenus par ultrafiltration simple ou après diafiltration étaient ensuite séchés par atomisation.

La figure 2 représente le réacteur enzymatique à membrane. Celui-ci comporte d'abord une cuve à réaction désignée par la référence générale 15. L'alimentation continue en protéines de lactosérum se fait par la canalisation 16. Un appareil 17 sert à la fois à mesurer et à maintenir le pH dans la cuve de réaction en neutralisant les ions $H^+$ libérés lors de la coupure des liaisons peptidiques. L'appareil était un pH stat Mettler comprenant un amplificateur de potentiel, un présélecteur du point d'équivalence et une burette automatique de distribution du réactif, ce dernier étant un composé basique tel que mentionné ci-dessus. Aucun encrassement excessif de l'électrode n'a été constaté. Le produit d'hydrolyse prélevé dans la cuve de réaction par la canalisation 18 est acheminé par une pompe pneumatique 19 à membrane. Un exemple pratique est la pompe modèle AMICON LP 20 A, capable de débiter 720 1/h à 1,7 bar. Au refoulement de la pompe, le produit passe dans une canalisation 20 et est amené sur un préfiltre 21 ayant une porosité de 150 microns. La référence 22 désigne le module d'ultrafiltration. Dans un exemple spécifique, on a utilisé le système DC 10 S de la Société AMICON comprenant des cartouches d'ultrafiltration à fibres creuses. Le perméat était récupéré dans une canalisation 23 et constituait l'hydrolysat peptidique recherché. Le rétentat du module 22 était prélevé par la canalisation 24, introduit sur un échangeur 25 et acheminé par la canalisation 26 pour être recyclé dans la cuve de réaction 15.

Les membranes utilisées étaient du type fibres creuses ayant les caractéristiques suivantes:

| Type | Seuil de coupure | Surface | Fabricant |
|---|---|---|---|
| H10 P 5 | 5000 | 0,9 m² | Amicon |
| H10 P 5 | 10000 | | |
| PM₂ | 2000 | 1,4 m² | Romicon |
| PM 50 | 50000 | | |

Pour les essais rapportés ci-après, on a mis en oeuvre un lactosérum d'origine industrielle résultant d'un mélange de lactosérum d'Emmental et de Saint Paulin, son pH variant entre 6,2 et 6,4. Après écrémage, le lactosérum était thermisé à 58—60°C pendant 15 secondes environ. Il pouvait alors être concentré par évaporation à un taux de matière sèche d'environ 50%. Ce préconcentré était alors dilué 2,5 fois pour éviter la cristallisation excessive du lactose et refroidi à une température proche de 4°C avant stockage pendant une nuit. En variante, certains lactosérums utilisés dans les exemples ci-après ont été préparés sur place à partir de laits crus ou thermisés par précipitation lactique ou présure.

Dans les conditions habituelles d'expérimentation, le concentré protéique était reconstitué à une teneur de 100 g/l en protéines dans la cuve de réaction 15. La température de réaction était fixée à 40°C environ. La solution de protéines était ajoutée dans la cuve 15. L'équipement 17 permet la mesure et le maintien du pH. Après équilibrage de la température et ajustement du pH, on a ajouté l'enzyme (pancréatine) reconstituée extemporanément. L'addition du composé alcalin était enregistrée en continu en vue du maintien du pH dans la zone de réaction. Chaque prélèvement était soumis à la précipitation dans l'acide trichloracétique à 12%. La protéolyse était suivie en déterminant sur le surnageant l'azote soluble et les groupements $NH_2$ libérés.

Les teneurs en substance sèche des échantillons de lactosérum, de rétentat, d'ultrafiltrat et des hydrolysats étaient déterminées par dessiccation à l'etuve à 102—105°C pendant 7 heures.

Les teneurs en azote étaient déterminées par microkjeldahl sur un autoanalyseur Technicon:
– les teneurs en azote total étaient obtenues par dosage direct après dilution (Matières Azotées Totales);
– les teneurs en azote non protéique (NPN) étaient déterminées par le dosage après précipitation des protéines dans l'acide trichloracétique à 12%.

Les teneurs en cendres étaient déterminées selon la méthode AOAC (Référence AOAC Standard, 1945).

La détermination des teneurs en substances minérales était réalisée par absorption atomique sur un appareil Technitron 1200 suivant le protocole de MURTHY, G. K., RHEA, V. ( 1967)—Determination of major cations in milk by atomic absorption spectrophotometry, J. Dairy Sc., 50, 313—317 modifié par BRULE et al. (1974)—Etude de la teneur en minéraux des produits obtenus lors de l'ultrafiltration du lait sur membrane. Le Lait. 539—540, 600—615.

Le dosage des groupements $NH_2$ libres était effectué par réaction à la ninhydrine après hydrolyse alcaline selon HIRS et al. (1956) J. Biol. Chem. 219 (1190) ou à l'aide de l'acide trinitrobenzène sulfonique selon FIELDS (1971) Biochem. J. 124, 581—590.

Le dosage des acides aminés était effectué selon la méthode de SPACKMAN et al. (1958) Anal. Chem. 30, 1190. Les peptides (2 mg) sont hydrolysés pendant 24 h, 48 h et 96 h à 110°C dans des tubes scellés sous vide par 1 ml de HCl 6N, distillé deux fois. Les résultats sont exprimés en g pour 100 g d'acides aminés, après

correction pour destruction pendant l'hydrolyse pour la thréonine, sérine, cystine et tyrosine.

Le tryptophane était dosé séparément selon la méthode de SPIES (1957) par réaction colorimétrique avec le paradiméthylaminobenzaldéhyde après hydrolyse par la pronase.

La mesure des densités optiques à 280 nm, à 420 nm (dosage des groupements $NH_2$ par le TNBS) à 570 nm (dosage des groupements $NH_2$ par la ninhydrine), ou le tracé des spectres d'absorption des protéines, des peptides ou des acides aminés dans le but de leur identification étaient effectués sur un spectrophotomètre Beckman type Acta VI, en cellule de quartz d'un centimètre de chemin optique.

Le dosage de l'activité résiduelle de la trypsine dans les hydrolysats peptidiques était effectué selon la méthode de HUMMEL (1959) Can. J. Biochem. Physiol., 37, 1393 en suivant l'augmentation de l'absorption à 247 nm, due à l'hydrolyse d'une substance synthétique, le p-toluènesulfonyl-L-arginine méthyl ester (TAME).

La détermination qualitative et quantitative des protéines solubles (lactoglobuline, lactalbumine, sérum albumine) dans le lactosérum, les rétentats, les ultrafiltrats, les concentrés protéiques avant, pendant et après hydrolyse, était obtenue par électrophorèse en gel de polyacrylamide selon la méthode d'ASCHAFFENBURG (1964)—Protein phenotyping by direct polyacrylamide gel electrophoresis of whole milk. Biochem. Biophys. Acta, 82, 188—191, modifiée: le gel utilisé était soit à 5% soit à 9% d'un mélange 1:1 d'acrylamide et de bisacrylamide (Cyanogum 41: Apelab Paris).

Les plaques, après révélation au noir amido et décoloration à l'acide acétique 10%, étaient lues à l'aide d'un densitomètre Philips.

La séparation des protéines, des peptides et acides aminés libres selon leur poids moléculaire était obtenue par filtration sur gels de Séphadex (Pharmacia).

En fonction de la nature des constituants à séparer, on a utilisé les gels suivants:

| Constituants à séparer | Nature du gel | Eluant |
|---|---|---|
| Protéines-protéines | $G_{100}$ | Tampon phosphate pH 7,5 |
| Protéines-gros peptides | $G_{75}$ | Tampon phosphate pH 7,5 |
| Peptides-peptides | $G_{50}$, $G_{25}$ | Acide acétique 30% |
| Peptides-acides aminés libres | $G_{15}$ | Acide acétique 30% |

Les éluats étaient analysés par mesure de la densité optique à 280 nm et par dosage des groupes $NH_2$ libres par la ninhydrine.

Le fractionnement des peptides et acides aminés en fonction de leur point isoélectrique et de leur charge a été obtenu grâce à l'utilisation des résines échangeuses d'ions type cationique (Ag 50xW₂—Laboratoire Biorad, Richmond Californie). L'hydrolysat peptidique était mis en présence de la résine en discontinu à différents pH. Après un certain temps de contact et décantation, le surnageant était analysé.

Exemple 1

Dans cet exemple, on a utilisé un dispositif du type général représenté à la figure 1 et un réacteur enzymatique du type représenté à la figure 2. Le lactosérum était triaté au préalable dans un séparateur centrifuge 3 à la vitesse de 1000 g pendant 15 minutes avant son entrée dans le dispositif.

L'enzyme utilisée était la pancréatine disponible sur le marché sous la dénomination "SIGMA", d'origine bovine ayant une activité de 4 NF. Les conditions réactionnelles dans la cuve 15 du réacteur enzymatique étaient les suivantes:
- température 40°C
- pH maintenu à 8,0
- teneur en protéines de la solution alimentant le réacteur: 80 g/l
- rapport pancréatine/substrat: 12%

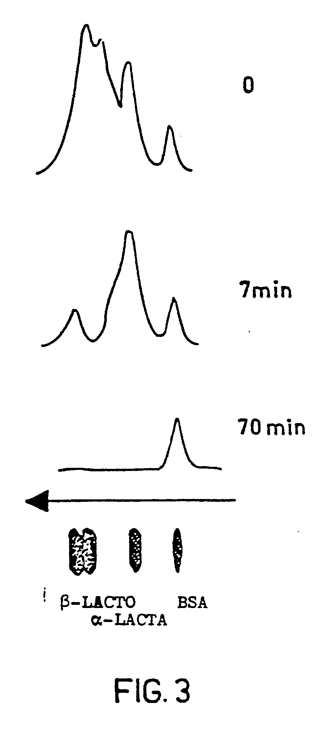

L'hydrolyse des protéines de lactosérum a été suivie par électrophorèse en gel de polyacylamide. Les résultats sont illustrés à la figure 3. Cette figure montre à différents stades de la réaction, aux temps respectivement 0, 7 minutes et 70 minutes, l'allure des courbes de l'électrophorèse correspondant à la solubilisation des protéines de lactosérum. Le diagramme à la partie inférieure de la figure 3 représente la migration des diverses protéines. La β-lactoglobuline est représentée par l'abréviation β-lacto, l'alphalactalbumine par l'abréviation α-lacta et la sérum albumine bovine par l'abréviation BSA.

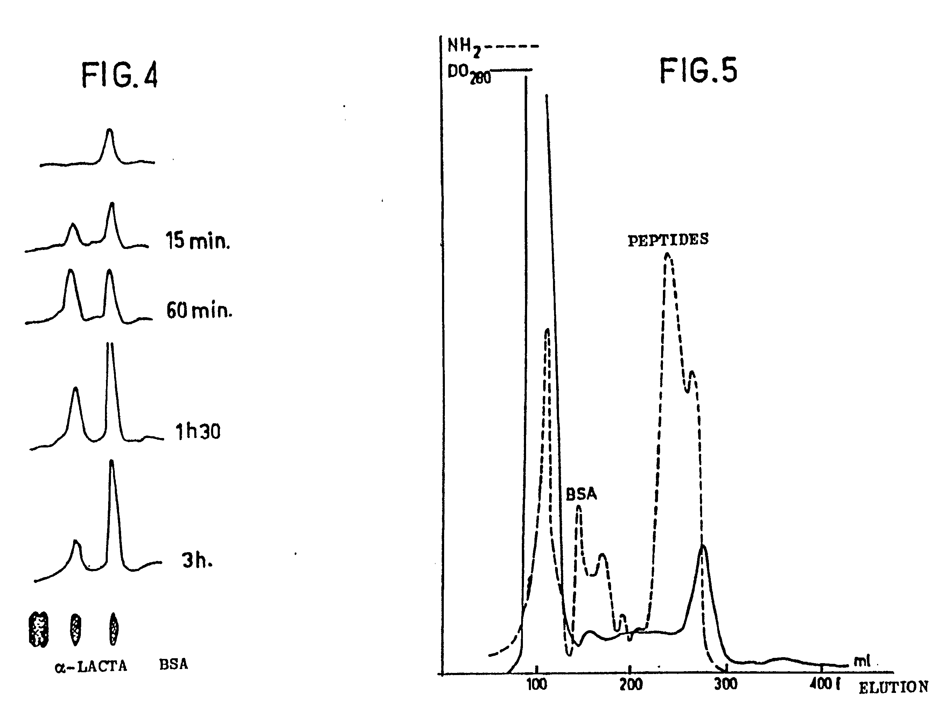

Le gel de polyacrylamide peut être utilisé à une concentration de 9%. Les résultats représentés à la figure 3 montrent que la vitesse d'hydrolyse des protéines est différente. La β-lactoglobuline est plus rapidement hydrolysée que l'α-lactalbumine et elle même que la sérumalbumine. Cette dernière est au moins partiellement insensible à la protéolyse. La sérum albumine bovine du lactosérum ne peut donc être hydrolysée totalement. La figure 4 est une figure analogue à celle de la figure 3 mais apportant des résultats sur le fonctionnement continu du réacteur. Les mesures par électrophorèse sur gel de polyacrylamide à 9% de l'hydrolyse en continu du concentrat de protéines de lactosérum par la pancréatine en réacteur enzymatique confirment que la sérumalbumine bovine s'accumule au niveau du réacteur.

Après 3 heures de fonctionnement du réacteur, le pied de cuve a été purifié par diafiltration et analysé. Les résultats sont représentés sur le diagramme de la figure 5. Cette figure 5 représente la répartition moléculaire des constituants présents dans le réacteur enzymatique en fin d'hydrolyse pancréatique des concentrats protéiques de lactosérum après purification par diafiltration (Séphadex G—100—tampon phosphate; pH 7,5). Le profil de filtration indique la présence de trois groupes de constituants:

– le premier, de poids moléculaire le plus élevé, présentant une absorption à 280 nm forte (présence d'un louche) pour une teneur en $NH_2$ faible,

– le second, dont l'image électrophorétique et le spectre d'absorption à 280 nm correspondent à ceux de la sérum albumine bovine,

– le troisième, de faible poids moléculaire: les peptides.

L'analyse chimique globale montre par ailleurs la présence de matières grasses qui y représentent prés de 50% de la matière sèche.

Exemples 2 et 3

Ces exemples concernent également la solubilisation en continu des protéines du lactosérum par la pancréatine en réacteur enzymatique. Les conditions de fonctionnement sont indiquées dans le tableau 1.

TABLEAU I

|  | Exemple 2 | Exemple 3 |
| --- | --- | --- |
| Seuil de coupure de la membrane | 5000 | 5000 |
| Température | 40°C | 37°C |
| pH | 8,5 | 8,5 |
| Teneur en protéines initiale | 84,4 g/l | 82 g/l |
| Base utilisée | KOH 2N | KOH 2N |
| Rapport enzyme/substrat | 11,8 % | 12,6% |
| Durée préhydrolyse | 2 heures | 1 h 30 |
| Teneur en protéines de la solution alimentant le réacteur | 45 g/l | 50 g/l |

Dans les deux essais, le taux d'hydrolyse apparent (azote soluble/azote total) dans le perméat a été maintenu constant grâce à un apport faible d'enzyme. La durée de fonctionnement du réacteur était de 7 à 8 heures.

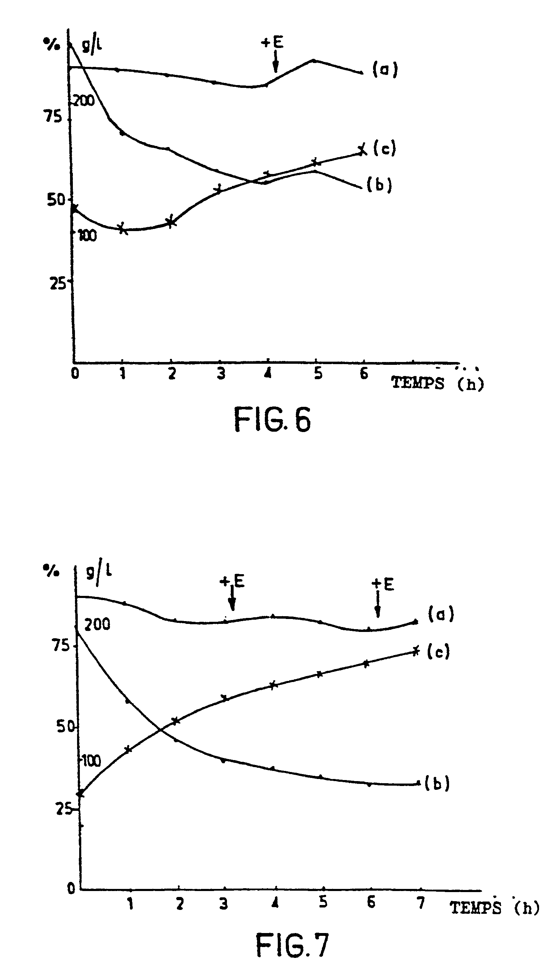

Les résultats apparaissent le mieux sur les diagrammes des figures 6 et 7. On a porté en abscisses le temps de réaction en heures et en ordonnées les concentrations en matières sèches ou en protéines des produits considérés en g/l. Aux figures 6 et 7 les courbes a) et b) représentent respectivement le taux d'hydrolyse apparent du perméat et du rétentat d'ultrafiltration tandis que la courbe c) représente l'extrait sec du rétentat.

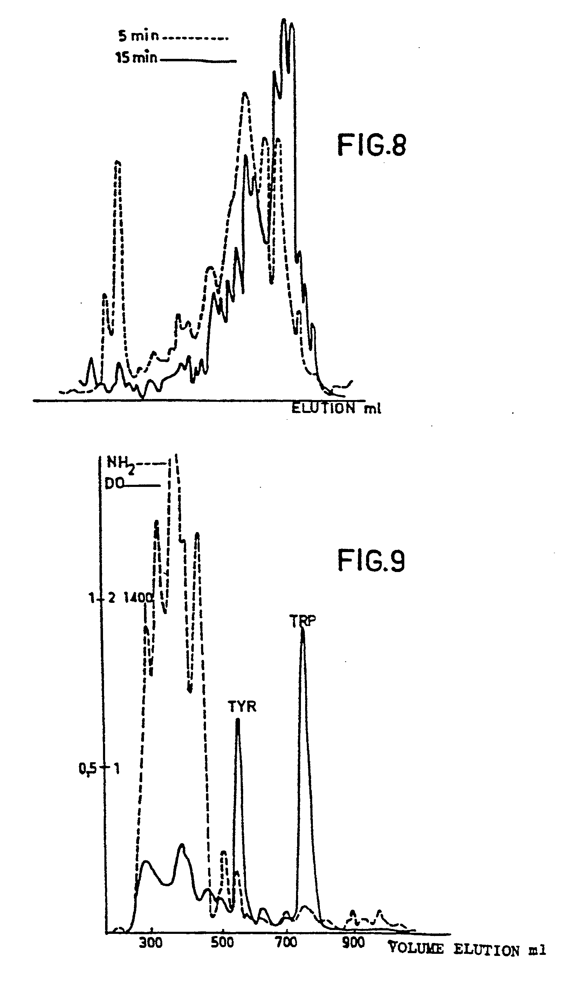

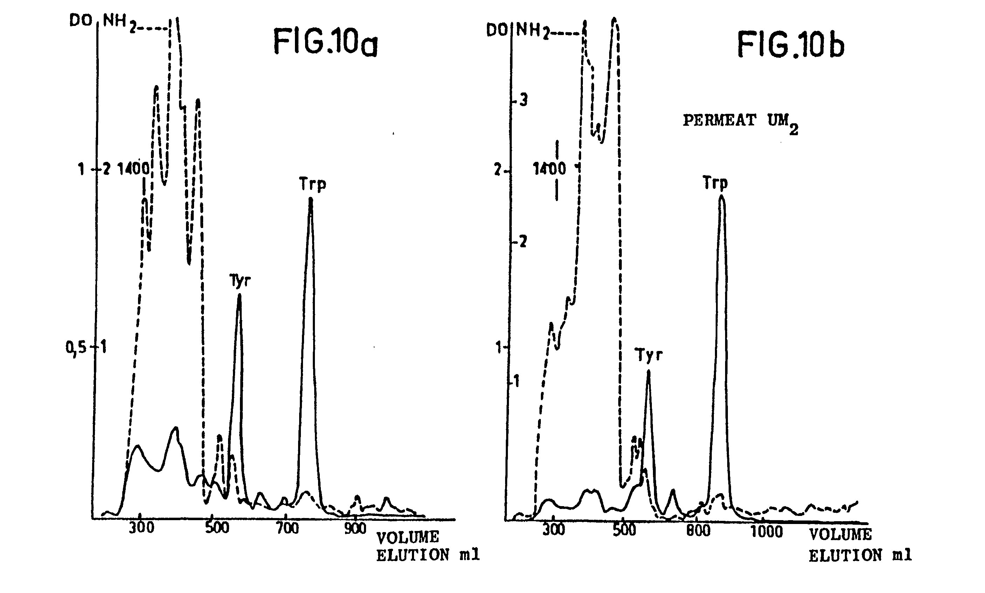

On a procédé ensuite à la caractérisation des hydrolysats obtenus. La figure 8 illustre la répartition moléculaire des peptides obtenus par hydrolyse pancréatique d'un concentré protéique de lactosérum en fonction du temps de contact enzyme/substrat. Les mesures ont été effectuées par filtration sur gel Sephadex G 50 et avec élution à l'acide acétique à 30%. La figure 8 met en évidence l'influence du temps de contact enzyme/substrat. La figure 9 illustre la répartition moléculaire des peptides obtenus par hydrolyse pancréatique d'un concentrat de protéine de lactosérum après hydrolyse totale en réacteur enzymatique, les mesures ayant été faites sur gel SEPHADEX G 15 (élution à l'acide acétique à 30%). Comme l'illustre la figure 9, tous les peptides obtenus ont une taille inférieure à 2000, la majorité se situant entre le dipeptide et le décapeptide. Le taux d'acides aminés libres est faible et peut être évalué à un taux inférieur à 10%. L'utilisation de diverses membranes présentant des seuils de coupure variés montre qu'il n'existe pas de différence entre les peptides obtenus sur membranes à seuil de coupure 5000 et ceux obtenus sur membranes à seuil de coupure 10.000. La figure 10 montre que le passage d'un hydrolysat pancréatique, obtenu sur membrane à seuil de coupure 5000, sur une membrane à seuil de coupure 15000 modifie le profil de répartition moléculaire des peptides. Cette figure illustre donc la possibilité de modifier la répartition des peptides en soumettant l'hydrolysat initial total obtenu préalablement sur membrane 5000 à une ultrafiltration sur membrane PM2 à seuil de coupure 1500—2000. Les mesures ont été faites sur gel Séphadex G 15 (élution à l'acide acétique à 30%.

On a effectué d'autres mesures sur la composition des produits obtenus en déterminant celles des fractions non dialysables et dialysables. La coupure par la dialyse se situe aux environs du poids moléculaire 2000. Le tableau 2 ci-après correspond aux aminogrammes des fractions dialysables I et non dialysables II d'un hydrolysat pancréatique de concentré protéique de lactosérum.

TABLEAU 2

| | I* | II* |
|---|---|---|
| Ile | 7,0 | 5,70 |
| Leu | 10,96 | 7,52 |
| Lys | 8,92 | 9,10 |
| Met | 2,20 | 2,10 |
| Cys+Cyst | 2,0 | 3,70 |
| Phe | 3,63 | 1,91 |
| Tyr | 3,03 | 5,80 |
| Thr | 6,70 | 6,70 |
| Trp | 1,62 | 1,13 |
| Val | 6,20 | 5,40 |
| Arg | 2,72 | 2,17 |
| His | 1,98 | 1,34 |
| Ala | 5,16 | 4,19 |
| Asp | 11,07 | 11,80 |
| Glu | 15,82 | 20,21 |
| Gly | 1,92 | 1,95 |
| Pro | 5,98 | 8,96 |
| Ser | 4,70 | 5,10 |

*I— Hydrolysat pancréatique de protéines de lactosérum (ler ultrafiltrat) partie dialysable.
**II—Hydrolysat pancréatique de protéines de lactosérum (ler ultrafiltrat) partie non dialysable.

Les résultats sont exprimés en g/100 g de protéines sauf pour le tryptophane (g/100 g de poudre lyophilisée).

Pour caractériser encore davantage l'hydrolysat pancréatique, on a mis celui-ci en présence d'une résine cationique à raison de 7 g de résine par 100 ml d'hydrolysat ayant une teneur de 36 g/l de peptides. La mise en contact a été réalisée pendant deux heures à la température ambiante et à divers PH.

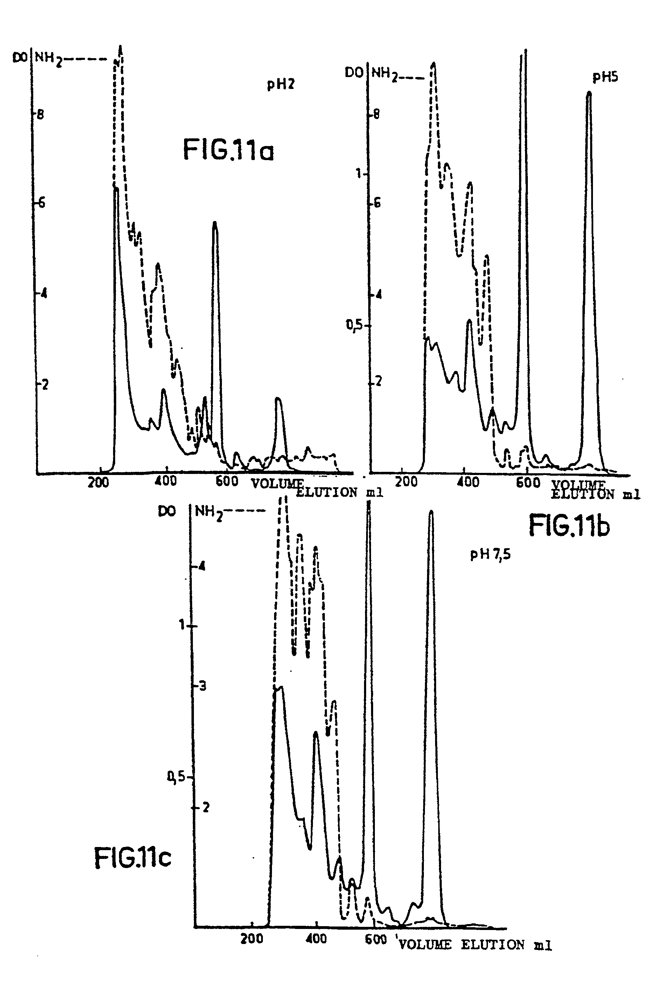

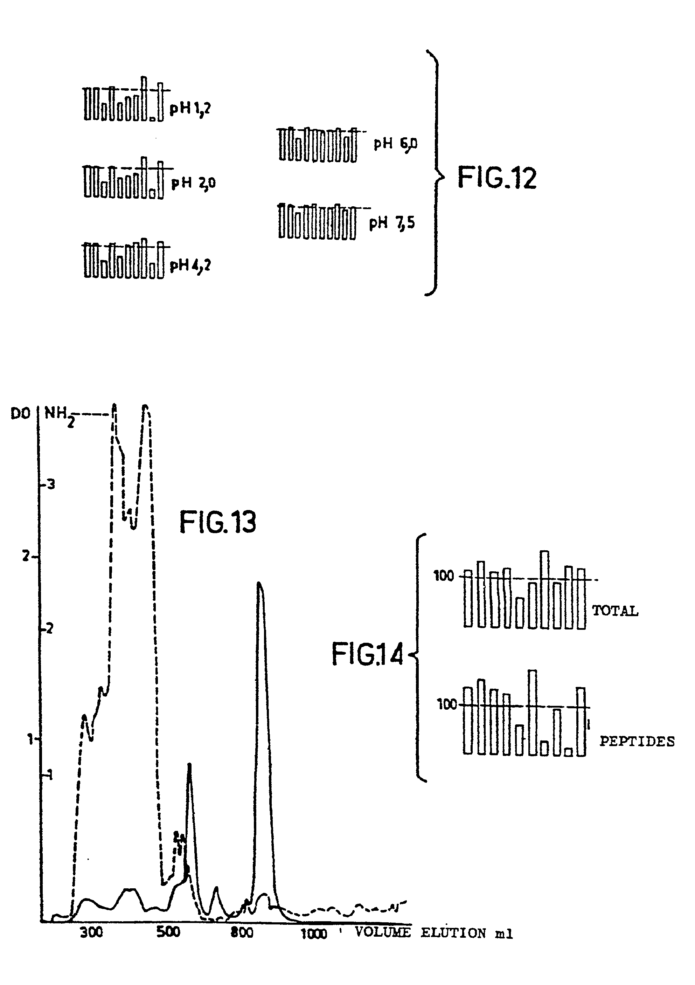

Les figures 11a, 11b, et 11c montrent la répartition moléculaire des peptides non fixés sur la résine cationique contenus dans les différents surnageants respectivements à pH 2, pH 5 et pH 7,5. Les diagrammes représentes à la figure 12 indiquent leur composition en acides aminés essentiels (exprimés en % de l'hydrolysat initial). La séquence des acides aminés sur les diagrammes est, de gauche à droite, la suivante: Ile, Leu, Lys, Met, Cys, Phe, Tyr, Thr, Trp et Val.

On observe une fixation différentielle des peptides suivant le pH de la réaction. A pH 2, les petits peptides et acides aminés libres se fixent préférentiellement aux plus gros. Ces derniers sont appauvris en lysine, cystéine, tryosine, phénylalanine et tryptophane. Lorsque le pH se rapproche de la neutralité, la fixation d'azote diminue. A pH 7,5 la composition en acides aminés du surnageant est très voisine de celle de l'hydrolysat initial.

Ces différents résultats montrent qu'un fractionnement des hydrolysats est possible, selon cette technique. En variant le pH de la réaction, la nature des résines, leur concentration, il est possible d'obtenir des fractions peptidiques à caractéristiques précises.

On a effectué d'autres expériences en ultrafiltrant l'hydrolysat total sur des membranes à seuil de coupure égal à 2000 environ, qui sont capables de retenir partiellement les peptides de poids moléculaire supérieur à 1500. La figure 13 illustre la répartition moléculaire des peptides et acides aminés libres contenus dans la fraction non retenue par la membrane 2000 (gel Sephadex G 15-acide acétique 30%). La fraction de l'hydrolysat pancréatique non retenu est appauvrie en cystéine et enrichie en phénylalanine, tryptophen, par rapport à l'hydrolysat d'origine. La figure 14 illustre la composition en acides aminés essentiels (exprimée en % de leur teneur dans l'hydrolysat initial) des peptides et acides aminés libres contenus dans la fraction non retenue par la membrane 2000.

Exemple 4

On a opéré dans des conditions similaires à celles des exemples 2 et 3. L'hydrolysat peptidique a été concentré puis séché. La poudre avait les caractéristiques définies ci-après

| Analyse chimique | 100 g |
|---|---|
| Matière séche | 96,3 |
| Matières azotés totales | 72,0* |
| | 74,2** |
| | 76,6*** |
| Cendres | 11,0 |
| Sucres réducteurs | 0 |
| Matières grasses | 0 |
| Minéraux Ca | 0,94 |
| Na | 0,30 |
| K | 4,1 |

Les analyses de la teneur en azote ont été faites par microkjeldahl. La dilution de la poudre ayant été faite dans

*citrate de sodium 2%
**NaOH 0,5 N
***eau distillée.

Les résultats sont exprimés en azotex6,38.
– précipitation au TCA 12% =:nulle
– dosage NPN surnageant N/100 poudre 11g.

| Aminogramme | | | |
|---|---|---|---|
| Ile | 6,0 | Arg | 2,7 |
| Leu | 9,9 | His | 1,7 |
| Lys | 9,2 | Ala | 4,9 |
| Cys | 1,8 | Asp | 9,5 |
| Phe | 3.2 | Glu | 17,6 |
| Thr | 6,7 | Gly | 1,7 |
| Tyr | 3,6 | Pro | 6,2 |
| Trp | 2,0 | Ser | 5,5 |
| Val | 5,5 | Met | 2,0 |

Les exemples qui suivent illustrent l'application des produits de l'invention dans des compositions constituant des aliments de réanimation.

Exemple 5

Cet exemple concerne un produit de réanimation utilisable par voie entérale chez des malades nécessitant un apport protéique de l'ordre de 7 à 12% de l'apport calorique total. Un tel produit répond à des besoins nutritionnels en cas de mucoviscidose ou fibrose kystique du pancréas, d'insuffisance rénale, ainsi que pour des malades présentant une atteinte infectieuse ou inflammatoire de la paroi intestinale. Ces protéines sont amenées de préférence sous forme pré-digérée.

Une composition centésimale appropriée est la suivante

| | | |
|---|---|---|
| — hydrolysat peptidique selon l'invention | | 2,50 g |
| — lipides: | | |
| mélange à parties égales de: | | |
| — huile de beurre | 0,5 g | |
| — T.C.M. | 0,5 g | |
| — huile de mais | 0,5 g | 4,10 g |
| — huile de trounesol | 0,5 g | |
| monostéarate de glycérol | 2,1 g | |
| — glucides | | |
| — polymères de glucose | 10 g | |
| — glucose | 1,5 g | 13,00 g |
| — galactose | 1,5 g | |
| — vitamines | | |
| A, D, E, $B_1$, $B_2$, PP, $B_5$, $B_6$ | | selon les recomman- |
| $B_{12}$, acide folique, biotine | | dations FAO/OMS |
| — éléments minéraux | | 0 455 g |

(calcium, sodium, potassium, magnésium, phosphore, zinc, fer, cuivre, manganèse, chlore, iode).

| | |
|---|---|
| — eau distillée | q.s.p. 100 |

## Exemple 6

Cet exemple concerne un produit de réanimation utilisable par voie entérale chez des malades nécessitant un apport protéique de l'ordre de 25% de l'apport calorique total, sous forme prédigérée et un apport lipidique faible. Il peut en être ainsi en cas de destruction tissulaire importante après grands traumatismes et de brûlures.

Une composition centésimale appropriée est la suivante:

| | | |
|---|---|---|
| — hydrolysat peptidiques selon l'invention | | 8 g |
| —lipides: | | |
| —T.C.M. | 2,30 g | |
| —huile très riche en acides gras essentiels | 0,50 g | 2,90 g |
| —émulsifiant | 0,10 g | |
| —glucides: | | |
| —petits polymèras de glucose | 12,7 g | |
| —glucose | 3 g | 18,7 g |
| —galactose | 3 g | |
| —vitamines | | |
| —A, D, E, $B_1$, $B_2$, PP, $B_5$, $B_6$ | | selon les recomman- |
| $B_{12}$, acide folique, biotine | | dations FAO/OMS |
| vitamine C | | |
| — éléments minéraux | | |
| — (calcium, sodium, potassium, magnésium, phosphore, zinc, fer, cuivre, manganèse, chlore, iode) | | 0,455 g |
| — eau distillée | | q.s.p. 100 |

## Exemple 7

Cet exemple illustre la préparation de compositions pharmaceutiques pour l'administration par voie orale. Des comprimés ont été préparés de la manière usuelle à partir de la formulation suivante:

| | |
|---|---|
| hydrolysat peptidique selon l'invention | 200 mg |
| excipient QS pour un comprimé terminé à | 300 mg |

L'excipient utilisé était le talc, le stéarate de magnésium ou la silice disponible sur le marché sous la dénomination "aérosil".

On a préparé de la même manière des gélules dosés à 100 mg d'hydrolysat peptidique selon l'invention et contenant un excipient usuel QS pour un gélule terminé à 200 mg.

## Revendications

1. <u>Hydrolysat enzymatique total</u> de protéines de lactosérum contenant des peptides et capable d'être directement assimilable par la muqueuse intestinale, caractérisé en ce qu'il ne contient pas de protéines résiduelles, en ce qu'au moins 50 % des peptides possèdent 2 à 5 acides aminés et en ce que la proportion d'acides aminés libres est inférieure à 15 %.

2. Hydrolysat selon la revendication 1, caractérisé en ce qu'il contient 70 à 90 % de l'azote présent sous forme de peptides ayant un nombre d'acides aminés inférieur à 10.

3. Hydrolysat selon l'une des revendications 1 ou 2, ne contenant aucune fraction précipitable à l'acide trichloracétique à 12 % et répondant à l'aminogramme ci-après, les chiffres suivants étant exprimés en gramme d'acide aminé pour 100 g d'acides aminés :

### Aminogramme

| Ile | 6,0 | Arg | 2,7 |
|-----|-----|-----|-----|
| Leu | 9,9 | His | 1,7 |
| Lys | 9,2 | Ala | 4,9 |
| Cys | 1,8 | Asp | 9,5 |
| Phe | 3,2 | Glu | 17,6 |
| Thr | 6,7 | Gly | 1,7 |
| Tyr | 3,6 | Pro | 6,2 |
| Trp | 2,0 | Ser | 5,5 |
| Val | 5,5 | Met | 2,0 |

4. Procédé pour l'obtention d'un hydrolysat selon l'une quelconque des revendications 1 à 3, dans lequel on soumet un concentré protéique de lactosérum à une hydrolyse enzymatique continue avec une enzyme protéolytique capable de reconstituer la digestion protéique qui prend place in vivo dans l'organisme humain, en introduisant ce concentré avec l'enzyme dans une zone de réaction, on soutire en continu le produit de réaction en l'amenant de la zone de réaction dans une zone d'ultrafiltration, de laquelle on soutire également en continu un perméat qui représente l'hydrolysat peptidique constituant le produit recherché, la réaction d'hydrolyse étant poursuivie jusqu'à ce que le produit ne contienne plus de protéines résiduelles, c'est-à-dire ne présente aucun azote précipitable par l'acide trichloracétique à 12%, et jusqu'à obtention d'un hydrolysat peptidique dont au moins 50% des peptides contiennent 2 à 5 acides aminés, un recyclage pouvant être prévu de la zone d'ultrafiltration jusqu'à la zone de réaction.

5. procédé selon la revendication 4, caractérisé en ce qu'on soumet à l'hydrolyse enzymatique un rétentat d'ultrafiltration du lactosérum.

6. procédé selon l'une des revendications 4 ou 5, caractérisé en ce qu'on prépare un concentré protéique de pureté élevée en soumettant le lactosérum à une ultrafiltration combinée avec une diafiltration.

7. procédé selon l'une des revendications 5 ou 6, caractérisé en ce que, préalablement à l'ultrafiltration, on soumet le lactosérum à une centrifugation pour éliminer les fractions insolubles.

8. procédé selon l'une quelconque des revendications 4 à 7, caractérisé en ce que, après obtention des protéines de lactosérum, on soumet le concentré protéique à une microfiltration pour en éliminer les fractions insolubles et les micro-organismes.

9. procédé selon l'une quelconque des revendications 4 à 8, caractérisé en ce que, à titre d'enzyme, on utilise une enzyme capable de digérer totalement les protéines du lactosérum dans les conditions de la digestion d'un organisme humain, ladite enzyme étant la pancréatine sous forme d'extrait pancréatique naturel ou un mélange synthétique, à base de trypsine et de chymotrypsine, avec d'autres enzymes secondaires, dont la composition se rapproche de celle de l'extrait naturel .

10. Procédé selon l'une quelconque des revendications 4 à 9, caractérisé en ce qu'on ajuste le pH dans la zone de réaction à une valeur comprise entre 7 et 9, de préférence de 7,5 à 8,5 par addition d'un composé

à réaction basique tel que l'hydroxyde ou le carbonate de sodium, l'hydroyde ou le carbonate de potassium, l'hydroxyde de calcium, l'hydroxyde d'ammonium ou un mélange desdits composés.

11. Procédé selon l'une quelconque des revendications 4 à 10, caractérisé en ce qu'on opère l'hydrolyse à une température de 37 à 45°C, en particulier de 40°C environ.

12. Procédé selon l'une quelconque des revendications 4 à 11, caractérisé en ce qu'on utilise des rapports enzyme/substrat de 8 à 15% en poids, en particulier de l'ordre de 12%.

13. Procédé selon l'une quelconque des revendications 4 à 12, caractérisé en ce que, dans le réacteur enzymatique à membrane, on utilise des membranes capables de retenir efficacement l'enzyme.

14. Application de l'hydrolysat selon l'une quelconque des revendications 1 à 3 comme aliment ou supplément alimentaire répondant à des besoins spécifiques nutritionnels.

15. Aliment diététique conforme à l'application selon la revendication 14, dans lequel l'hydrolysat est associé à un véhicule neutre convenant à l'alimentation orale ou entérale.

16. Aliment de réanimation conforme à l'application selon la revendication 14, dans lequel l'hydrolysat est associé à un véhicule neutre convenant à l'alimentation orale ou entérale.

17. Produit de nutrition thérapeutique conforme à l'application selon la revendication 15.

18. Médicament contenant l'hydrolysat selon l'une quelconque des revendications 1 à 3.

19. Composition pharmaceutique contenant l'hydrolysat selon l'une quelconque des revendications 1 à 3 en association avec un véhicule neutre usuel convenant à l'administration orale ou entérale.

20. Application de l'hydrolysat selon l'une quelconque des revendications 1 à 3 ou de la composition selon la revendication 19 pour obtenir un médicament.


## Patentansprüche

1. Gesamt-Enzymhydrolysat von Laktoserum-Proteinen, enthaltend Peptide und befähigt, direkt über die Darmschleimhaut aufgenommen zu werden, dadurch gekennzeichnet, daß es keine Rest-Proteine enthält, daß wenigstens 50% der Peptide 2 bis 5 Aminosäuren aufweisen, und daß der Anteil an freien Aminosäuren unter 15% liegt.

2. Hydrolysat nach Anspruch 1, dadurch gekennzeichnet, daß es 70 bis 90% des vorhandenen Stickstoffs in Form von Peptiden enthält, die eine Aminosäure-Zahl von weniger als 10 aufweisen.

3. Hydrolysat nach einem der Ansprüche 1 oder 2, enthaltend keinerlei Fraktion, die mit 12% Trichloressigsäure fällbar ist, und entsprechend dem nachstehenden Aminogramm, wobei die folgenden Zahlen in Gramm Aminosäure pro 100 g Aminosäuren ausgedrückt sind:


## Aminogramm

| Ile | 6,0 | Arg | 2,7 |
| Leu | 9,9 | His | 1,7 |
| Lys | 9,2 | Ala | 4,9 |
| Cys | 1,8 | Asp | 9,5 |
| Phe | 3,2 | Glu | 17,6 |
| Thr | 6,7 | Gly | 1,7 |
| Tyr | 3,6 | Pro | 6,2 |
| Trp | 2,0 | Ser | 5,5 |
| Val | 5,5 | Met | 2,0 |


4. Verfahren zur Gewinnung eines Hydrolysats nach irgendeinem der Ansprüche 1 bis 3, wobei man ein Laktoserum-Proteinkonzentrat einer kontinuierlichen enzymatischen Hydrolyse mit einem proteolytischen Enzym unterwirft, das befähigt ist, die in vivo stattfindende Protein-Verdauung im menschlichen Organismus nachzuahmen, wobei das Konzentrat mit dem Enzym in eine Reaktionszone eingebracht wird, man das Reaktionsprodukt kontinuierlich abzieht, indem es von der Reaktionszone in eine Ultrafiltrationszone überführt wird, aus der ebenfalls kontinuierlich ein Filtrat abgezogen wird, welches das Peptid-Hydrolysat darstellt, das das gewünschte Produkt bildet, wobei die Hydrolyse-Reaktion fortgeführt wird, bis das Produkt keine Rest-Proteine mehr enthält, d.h., keinerlei Stickstoff mehr vorhanden ist, der sich durch 12% Trichloressigsäure ausfällen läßt, und bis zur Gewinnung eines Peptid-Hydrolysats, worin wenigstens 50% der Peptide 2 bis 5 Aminosäuren enthalten, wobei eine Rückführung von der Ultrafiltrationszone bis zur Reaktionszone vorgesehen sein kann.

5. Verfahren nach Anspruch 4, dadurch gekennzeichnet, daß ein Rückstand der Ultrafiltration des Laktoserums der enzymatischen Hydrolyse unterworfen wird.

6. Verfahren nach einem der Ansprüche 4 oder 5, dadurch gekennzeichnet, daß ein Protein-Konzentrat hoher Reinheit hergestellt wird, indem ein Laktoserum einer mit einer Diafiltration kombinierten Ultrafiltration unterworfen wird.

7. Verfahren nach einem der Ansprüche 5 oder 6, dadurch gekennzeichnet, daß das Laktoserum vor der Ultrafiltration zentrifugiert wird, um die unlöslichen Fraktionen zu entfernen.

8. Verfahren nach irgendeinem der Ansprüche 4 bis 7, dadurch gekennzeichnet, daß nach Gewinnung der Laktoserum-Proteine das Protein-Konzentrat einer Mikrofiltration unterworfen wird, um die unlöslichen Fraktionen und die Mikroorganismen daraus zu entfernen.

9. Verfahren nach irgendeinem der Ansprüche 4 bis 8, dadurch gekennzeichnet, daß als Enzym ein Enzym verwendet wird, das die Proteine des Laktoserums unter den Bedingungen der Verdauung eines menschlichen Organismus vollständig zu verdauen vermag, wobei das Enzym Pankreatin in Form von natürlichem Pankreas-Extrakt oder ein synthetisches Gemisch auf der Grundlage von Trypsin und Chymotrypsin mit anderen sekundären Enzymen ist, dessen Zusammensetzung der des natürlichen Extraktes nahekommt.

10. Verfahren nach irgendeinem der Ansprüche 4 bis 9, dadurch gekennzeichnet, daß der pH in der Reaktionszone durch Zugabe einer basisch reagierenden Verbindung wie etwa Natriumhydroxid oder -carbonat, Kaliumhydroxid oder -carbonat, Calciumhydroxid, Ammoniumhydroxid oder eines Gemischs der genannten Verbindungen auf einen Wert zwischen 7 und 9 eingestellt wird, vorzugsweise 7,5 bis 8,5.

11. Verfahren nach irgendeinem der Ansprüche 4 bis 10, dadurch gekennzeichnet, daß die Hydrolyse bei einer Temperatur von 37 bis 45°C, insbesondere bei etwa 40°C durchgeführt wird.

12. Verfahren nach irgendeinem der Ansprüche 4 bis 11, dadurch gekennzeichnet, daß Enzym/Substrat-Verhältnisse von 8 bis 15 Gew.-%, insbesondere in der Größenordnung von 12% angewandt werden.

13. Verfahren nach irgendeinem der Ansprüche 4 bis 12, dadurch gekennzeichnet, daß im Enzym-Membranreaktor Membranen verwendet werden, die das Enzym wirksam zurückzuhalten vermögen.

14. Verwendung des Hydrolysats nach irgendeinem der Ansprüche 1 bis 3 als Nahrungsmittel oder Nahrungsmittelergänzung, entsprechend speziellen Ernährungsbedürfnissen.

15. Diätetisches Nahrungsmittel gemäß Anwendung nach Anspruch 14, worin das Hydrolysat mit einem neutralen Träger kombiniert ist, der sich für die orale oder enterale Ernährung eignet.

16. Wiederbelebungsnahrungsmittel gemäß Anwendung nach Anspruch 14, worin das Hydrolysat mit einem neutralen Träger kombiniert ist, der sich für die orale oder enterale Ernährung eignet.

17. Therapeutisches Ernährungsprodukt gemäß Anwendung nach Anspruch 15.

18. Medikament, enthaltend das Hydrolysat nach irgendeinem der Ansprüche 1 bis 3.

19. Pharmazeutische Zusammensetzung, enthaltend das Hydrolysat nach irgendeinem der Ansprüche 1 bis 3 in Kombination mit einem neutralen Träger, der sich gewöhnlich für die orale oder enterale Verabreichung eignet.

20. Verwendung des Hydrolysats nach irgendeinem der Ansprüche 1 bis 3 oder der Zusammensetzung nach Anspruch 19 zur Gewinnung eines Medikaments.

## Claims

1. Total enzymatic hydrolysate from whey proteins, comprising peptides and capable of direct assimilation by the intestinal mucous membrane characterized in that it contains no residual proteins, in that at least, 50% of the peptides contain 2 to 5 amino acids, and in that the amount of free amino acids is less than 15%.

2. Hydrolysate according to claim 1, characterized in that it contains 70 to 90% of the nitrogen present in the form of peptides having a number of amino acids less than 10.

3. Hydrolysate according to one of claims 1 or 2, containing no fraction precipitable by 12% trichloroacetic acid and having the following aminogram, the following figures being expressed in g of amino acid per 100 g of amino-acids :

| Ile | 6.0 | Arg | 2.7 |
|-----|-----|-----|------|
| Leu | 9.9 | His | 1.7 |
| Lys | 9.2 | Ala | 4.9 |
| Cys | 1.8 | Asp | 9.5 |
| Phe | 3.2 | Glu | 17.6 |
| Thr | 6.7 | Gly | 1.7 |
| Tyr | 3.6 | Pro | 6.2 |
| Trp | 2.0 | Ser | 5.5 |
| Val | 5.5 | Met | 2.0 |

4. Process for obtaining a hydrolysate according to anyone of claims 1 to 3 wherein a whey protein concentrate is subjected to a continuous enzymatic hydrolysis with a proteolytic enzyme capable of simultating proteic digestion which takes place in vivo in the human body, by introducing said concentrate in a reaction zone with the enzyme, the reaction product is continuously drawn off by conducting it from the reaction zone in an ultrafiltration zone, from which a permeate is also continuously drawn off, which represents the peptidic hydrolysate constituting the desired product, the hydrolysis reaction being conducted until the product contains no more residual proteins, i. e. has no nitrogen precipitable by 12% trichloroacetic acid and until peptidic hydrolysate is obtained, at least 50% of the peptides of which contain 2 to 5 amino acids, a recycling step enventually being forseen from the ultrafiltration to the reaction zone.

5. Process according to claim 4, characterized in that an ultrafiltration whey retentate is submitted to the enzymatic hydrolysis.

6. Process according to one of claim 4 or 5, characterized in that a high purity protein concentrate is prepared by submitting the whey to an ultrafiltration combined with a diafiltration.

7. Process according to one of claim 5 or 6, charaterized in that, before the ultrafiltration step the whey is centrifugated to eliminate insoluble fractions.

8. Process according to anyone of claims 4 to 7 characterized in that, after obtention of whey proteins, the protein concentrate is subjected to a microfiltration in order to eliminate insoluble fractions and microorganisms.

9. Process according to anyone of claims 4 to 8 characterized in that, as enzyme, there is used an enzyme able to completely digest the whey proteins in the conditions of the digestion of body, said enzyme being pancreatine under the form of natural pancreatic extract or a synthetic mixture, containing trypsine or chymotrypsine with other secondary enzymes, the composition of which approximates the one of the natural extract.

10. Process according to anyone of claim 4 to 9, characterized in that in the reaction zone the pH is adjusted to a value comprised between 7 and 9, preferably 7.5 to 8.5 by addition of a basic reactive compound, such as sodium hydroxide or carbonate, potassium hydroxide or carbonate, calcium hydroxide, ammonium hydroxide or a mixture of said compounds.

11. Process according to anyone of claims 4 to 10, characterized in that the hydrolysis is carried out at a temperature between 37° and 45°C, in particular at about 40°C.

12. Process according to anyone of claim 4 to 11, characterized in that there are used enzyme/substrate ratios of 8 to 15% by weight, in particular of about 12%.

13. Process according to anyone of claims 4 to 12, characterized in that, in the enzyme reactor with membranes, membranes capable to effectively retain the enzyme are used.

14. Use of the hydrolysate according to anyone of claims 1 to 3, as food or food supplement adapted for specific nutritional requirements.

15. Diet food according to the use of claim 14, wherein the hydrolysate is associated with a neutral carrier suitable for enteral or oral feeding.

16. Care food according to the use of claim 14, wherein the hydrolysate is associated with a neutral carrier suitable for oral or enteral feeding.

17. Product for therapeutic nutrition in accordance with the use of claim 15.

18. Drug containing the hydrolysate according to anyone of claims 1 to 3.

19. Pharmaceutical composition containing the hydrolysate according to anyone of claim 1 to 3, in association with a conventional neutral carrier suitable for oral or enteral administration.

20. Use of the hydrolysate according to anyone of claims 1 to 3 or of the composition according to claim 19 for obtaining a drug.

LACTOSERUM

ULTRAFILTRATION

HYDROLYSAT

REACTEUR
ENZYMATIQUE

# FIG.1

# FIG.2

FIG. 3

# FIG.4

15 min.

60 min.

1h30

3h.

α-LACTA    BSA

# FIG.5

NH₂------
DO₂₈₀‾‾‾‾

PEPTIDES

BSA

100    200    300    400 ℓ
ml
ELUTION

22

EP 0 022 019 B2

FIG. 6

FIG. 7

FIG.8

FIG.9

FIG.10a

FIG.10b

PERMEAT UM₂

FIG.11a

FIG.11b

FIG.11c

pH 1,2

pH 2,0

pH 4,2

pH 6,0

pH 7,5

FIG.12

FIG.13

DO | NH₂

VOLUME ELUTION ml

FIG.14

TOTAL

PEPTIDES